Europäisches Patentamt

European Patent Office

Office européen des brevets

(19)

(11) Publication number: **0 068 838**
**B1**

(12) **EUROPEAN PATENT SPECIFICATION**

(45) Date of publication of patent specification: 17.09.86

(21) Application number: 82303318.8

(22) Date of filing: 25.06.82

(51) Int. Cl.⁴: **A 61 K 31/485** //
(A61K31/485,
31:19),(A61K31/485, 31:215)

(54) Analgesic process and composition.

(30) Priority: 26.06.81 US 277557
26.06.81 US 277558

(43) Date of publication of application:
05.01.83 Bulletin 83/01

(45) Publication of the grant of the patent:
17.09.86 Bulletin 86/38

(84) Designated Contracting States:
BE CH DE FR GB IT LI NL SE

(56) References cited:
DE-A-1 941 583

CHEMICAL ABSTRACTS, vol. 90, no. 5, January
29, 1979, abstract no. 33960z, page 39,
COLUMBUS OHIO (US)
Clinical Pharmacology and Therapeutics, vol.
27 (2), page 249, 1980

(73) Proprietor: THE UPJOHN COMPANY
301 Henrietta Street
Kalamazoo, Michigan 49001 (US)

(72) Inventor: Lomen, Pavel Luboslav
c/o The Upjohn Company 301 Henrietta Street
Kalamazoo Michigan 49001 (US)

(74) Representative: Perry, Robert Edward et al
GILL JENNINGS & EVERY 53-64 Chancery Lane
London WC2A 1HN (GB)

Courier Press, Leamington Spa, England.

EP 0 068 838 B1

## Description

This invention relates to the use of analgesics and to compositions for the management of pain by the administration of narcotic analgesics.

Narcotic analgesics have been used for the relief of moderate to severe pain. Severe pain, particularly, has required the use of narcotic analgesics in large and increasing dosage amounts. A disadvantage of the narcotic analgesics is the development of dependence or addiction and tolerance to their action. Further, adverse reactions, including respiratory and circulatory depression, are observed when large doses are used.

Ibuprofen and flurbiprofen are non-steroidal anti-inflammatory drugs. They have been used in rheumatic and degenerative diseases of the joints, for reducing platelet adhesiveness, and for dental pain.

Srivastava *et al.*, Clinical and Experimental Pharmacology and Physiology *5* (1978) 503—509, disclose that, among give prostaglandin synthesis inhibitors tested, ibuprofen inhibits the antinociceptive action of morphine. In tests to establish this, ibuprofen is administered to a rat, followed by morphine.

Cooper *et al*, abstracted in Clinical Pharmacology and Therapeutics *27* (2) (1980) 249, report the results of adminstering codeine, aspirin, ibuprofen, aspirin and codeine, or ibuprofen and codeine to subjects having moderate-to-severe post-operative pain following dental impaction surgery, and that the "ibuprofen-codeine combination was the most effective treatment for every analgesic parameter but was not statistically superior to ibuprofen alone".

A product according to the present invention contains flurbiprofen, or a $C_{1-8}$ alkyl ester or pharmacologically acceptable salt thereof, and a narcotic analgesic, as a combination preparation for simultaneous or separate use in the treatment of pain. It appears that such a combination can act synergically in the management of severe to moderate pain.

According to a second aspect of the invention, a composition comprises a narcotic analgesic and flurbiprofen, or a $C_{1-8}$ alkyl ester or pharmacologically acceptable salt thereof, usually together with a physiologically-acceptable excipient. A composition of the invention can be used to provide control of pain, while delaying or eliminating narcotic dependence and resistance.

The flurbiprofen may be used in the form of a salt such as an alkali metal, alkaline earth metal or ammonium salt. Alternatively, it may be used in the form of an alkyl ester, in any isomeric form, in which the alkyl group has from 1 to 8 carbon atoms.

Narcotic analgesics are, in general, well known. Suitable such compounds for use in the present invention are the naturally-occurring opium alkaloids and their semi-synthetic and synthetic derivatives. Examples are morphine, hydromorphone, oxymorphone, levorphanol, methadone, meperidine, anileridine, alphaprodine, fentanol, codeine, codone and oxycodone.

In the invention, the narcotic analgesic may be used in its usual dosage amount, with from 50 mg of flurbiprofen four or five times daily. After two days of administration, the dosage of narcotic analgesic may be lowered gradually over a period of 14 days, to the lowest acceptable amount of narcotic which maintains analgesia. After 14 days when the lowest narcotic amount is determined, the amount of flurbiprofen may be lowered to from 100 to 200 mg/day, to maintain the same degree of pain control.

The narcotic analgesic and flurbiprofen can be administered in the same dosage unit or can be prepared in separate dosage units, and the dosage units administered at the same time. Different forms of dosage units can be used, e.g. a tablet of flurbiprofen and an injection of narcotic.

The dosage forms for compositions of the present invention may be, for example, tablets, capsules, pills, powders granules, suppositories, sterile parenteral solutions or suspensions, sterile non-parenteral solutions or suspensions, or oral solutions or suspensions, for systemic administration. For oral administration, either solid or fluid dosage forms can be prepared.

Powders are prepared quite simply by comminuting the active ingredients to a suitably fine size and mixing with a similarly comminuted diluent. The diluent can be an edible carbohydrate material such as lactose or starch. Advantageously, a sweetening agent or sugar is present, as well as a flavouring oil.

Capsules are produced by preparing a powder mixture and filling into formed gelatin sheaths. Advantageously, as an adjuvant to the filling operation, a lubricant such as talc, magnesium stearate or calcium stearate is added to the powder mixture before the filling operation.

Soft gelatin capsules are prepared by machine encapsulation of a slurry of active ingredients with an acceptable vegetable oil, light liquid petrolatum or other inert oil or triglyceride.

Tablets are made by preparing a powder mixture, granulating or slugging, adding a lubricant and pressing into tablets. The powder mixture is prepared by mixing the active ingredients, suitably comminuted, with a diluent or base such as starch, lactose, kaolin or dicalcium phosphate. The powder mixture can be granulated by wetting with a binder such as corn syrup, gelating solution methylcellulose solution or acacia mucilage, and forcing through a screen. As an alternative to granulating, the powder mixture can be slugged, i.e. the mixture is run through a tablet machine and the imperfectly formed tablets which are produced broken into pieces. The slugs can be lubricated to prevent sticking to the tablet-forming dies by the addition of stearic acid, a stearate salt, talc or mineral oil. The lubricated mixture is then compressed into tablets.

Advantageously, the tablet can be provided with a protective coating consisting of a sealing coat or enteric coat of shellac, a coating of sugar and methylcellulose and a polish coating of carnauba wax.

Fluid dosage forms for oral administration can be prepared, such as syrups, elixirs and suspensions. Water-soluble forms can be dissolved in an aqueous vehicle together with sugar, flavouring agents and preservatives, to form a syrup. An elixir is prepared by using a hydroalcoholic vehicle with suitable sweeteners together with a flavouring agent. Suspensions of insoluble forms can be prepared with a suitable vehicle and a suspending agent such as acacia, tragacanth or methylcellulose.

For parenteral administration, fluid dosage forms are prepared, utilising the active ingredients and a sterile vehicle, water being preferred. The active ingredients, depending on the form and concentration used, can be either suspended or dissolved in the vehicle. In preparing solutions, the water-soluble active ingredients can be dissolved in water for injection and filter-sterilised before filling into a suitable vial or ampoule, and sealing. Advantageously, adjuvants such as a local anaesthetic, preservative and buffering agents can be dissolved in the vehicle. Co-solvents such as ethanol or propylene glycol can be used in the solvent system. Parenteral suspensions are prepared in substantially the same manner except that the active ingredients are suspended in the vehicle instead of being dissolved, and sterilisation cannot be accomplished by filtration. The active ingredients can be sterilised by exposure to ethylene oxide before suspension in the sterile vehicle. Advantageously, a surfactant or wetting agent is included in the composition, to facilitate uniform distribution of the active ingredients.

In addition to oral and parenteral administration, the rectal and vaginal routes can be utilised. Active ingredients can be administered by means of a suppository. A vehicle which has a melting point at about body temperature, or one that is readily soluble, can be utilised. For example, cocoa butter and various polyethylene glycols, e.g. the Carbowaxes, can serve as the vehicle.

Compositions for administration may be prepared in "unit dosage form", i.e. physically discrete units suitable as unitary dosages for human and animal subjects, each unit containing a predetermined quantity of active material calculated to produce the desired therapeutic effect in association with the required pharmaceutical diluent, carrier or vehicle. Examples of suitable unit dosages forms are tablets, capsules, troches, suppositories, powder packets, wafers, cachets, ampoules, vials, segregated multiples of any of the foregoing, and other forms as herein described.

The following Examples 1 to 3 illustrate compositions of the invention. Examples 4 and 5 illustrate the utility of products of the invention.

Example 1

Hard gelatin capsules

| Flurbiprofen | 50 g |
|---|---|
| Morphine sulfate | 15 g |
| Lactose | 100 g |
| Corn starch | 20 g |
| Talc | 20 g |
| Magnesium stearate | 2 g |

The flurbiprofen and morphine sulfate are finely divided by means of an air microniser; they are added to the other finely powdered ingredients and mixed thoroughly. The mixture is encapsulated in the usual manner, to give 1000 two-piece gelatin capsules for oral use, each capsule containing 50 mg flurbiprofen and 15 mg morphine sulfate.

The capsules are useful for preventing pain following laparotomy by the oral administration of one capsule four times a day. The narcotic dose may be reduced by using capsules containing morphine sulfate in a 7.5 or 3.75 mg amount, by substituting 7.5 or 3.75 g morphine sulfate for the 15 g given above.

Example 2
Soft gelatin capsules

One-piece soft gelatin capsules, for oral use, each containing 50 mg of flurbiprofen and 15 mg morphine sulfate (finely divided by means of an air microniser) are prepared by first suspending the compounds in 0.5 ml corn oil to render the material capsulatable, and then capsulating in the above manner.

The capsules are useful for preventing pain following the caesarian section by the oral administration of one capsule four times a day.

Example 3

Tablets

| Flurbiprofen micronised | 50 g |
|---|---|
| Morphine sulfate | 15 g |
| Lactose | 75 g |
| Corn starch | 50 g |
| Magnesium stearate | 4 g |
| Light liquid petrolatum | 5 g |

The flurbiprofen and morphine sulfate (finely divided by means of an air microniser) are added to the other ingredients and then thoroughly mixed and slugged. The slugs are broken down by forcing them through a 1.18 mm (number sixteen) screen. The resulting granules are then compressed into tablets, each tablet containing 50 mg of flurbiprofen and 15 mg morphine sulfate.

The tablets are useful for preventing pain following a broken femur by the oral administration of one tablet four times a day, for two days following setting the bone. The narcotic dose may be reduced by using tablets containing morphine sulfate in a 7.5 mg or 3.75 mg amount, by substituting 7.5 g or 3.75 g morphine sulfate for the 15 g given above.

Example 4

Aqueous suspension

| Flurbiprofen, aluminium salt, micronised | 20 g |
|---|---|
| Citric acid | 2 g |
| Benzoic acid | 1 g |
| Sucrose | 700 g |
| Tragacanth | 5 g |
| Lemon oil | 2 g |
| Deionised water, q.s. | 1000 ml |

The citric acid, benzoic acid, sucrose, tragacanth and lemon oil are dispersed in sufficient water to make 850 ml of suspension. The flurbiprofen aluminium salt (finely divided by means of an air microniser) is stirred into the syrup until uniformly distributed. Sufficient water is added to make 1000 ml.

The composition so prepared is useful for preventing pain of cancer of the bowels at a dose of one tablespoonful (15 ml) four times a day with 0.25 gram of morphine sulfate given I.M. four times a day.

Example 5
Aqueous solution

A sterile aqueous solution for parenteral (i.v.) injection is prepared by adding 350 mg flurbiprofen sodium salt to 1 litre sterile water for injection. Sterile containers are filled with the solution and sealed.

The solution is useful for preventing pain of inoperable cancer at a dose of one litre every 8 hours with 32 mg (0.5 grain) morphine sulfate every 8 hours.

In the Examples, an ester or salt, e.g. a methyl, ethyl, isopropyl or octyl ester or a sodium, potassium or ammonium salt, may be used instead of the flurbiprofen given in the Example. Further, instead of morphine, another narcotic analgesic may be used, as exemplified above.

## Claims

1. A product containing flurbiprofen, or a $C_{1-8}$ alkyl ester of pharmacologically acceptable salt thereof, and a narcotic analgesic as a combination preparation for simultaneous or separate use in the treatment of pain.

2. A composition which comprises a mixture of flurbiprofen, or a $C_{1-8}$ alkyl ester or a pharmacologically acceptable salt thereof, and a narcotic analgesic.

3. A composition according to claim 2, which additionally comprises a physiologically-acceptable excipient.

4. A product according to claim 1, in which the narcotic analgesic is selected from morphine, hydromorphone, oxymorphone, levorphanol, methadone, meperidine, anileridine, alphaprodine, fentanol, codeine, codone and oxycodone.

5. A composition according to claim 2 or claim 3, in which the narcotic analgesic is selected from those defined in claim 4.

## Patentansprüche

1. Ein Produkt, welches Flurbiprofen oder ein $C_{1-8}$ Alkyl-Ester oder dessen pharmakologisch akzeptables Salz und ein narkotisches Analgetikum enthält als Kombinationspräparat für die gleichzeitige oder gesonderte Behandlung von Schmerz.

2. Eine Verbindung aus einer Mischung von Flurbiprofen oder einem $C_{1-8}$ Alkyl-Ester oder dessen pharmakologisch akzeptablem Salz und einem narkotischen Analgetikum.

3. Ein Verbindung gemäß Anspruch 2, die zusätzlich einen physiologische akzeptablen Exzipienten enthält.

4. Ein Produkt gemäß Anspruch 1, in dem das narkotische Analgetikum gewählt wird aus Morphin, Hydromorphin, Oxymorphon, Levorphanol, Methadon, Meperidin, Anileridin, Alphaprodin, Fentanol, Codein, Codon und Oxycodon.

5. Ein Verbindung gemäß Anspruch 2 oder Anspruch 3, in der das narkotische Analgetikum aus den in Anspruch 4, aufgeführten Analgetika gewählt wird.

## Revendications

1. Produit contenant du flurbiprofène ou un ester d'alkyle en $C_1$ à $C_8$ ou un sel pharmacologiquement acceptable de ce composé et un analgésique narcotique, comme préparation en association pour l'usage simultané ou séparé dans le traitement de la douleur.

2. Composition qui comprend un mélange du flurbiprofène, d'un ester d'alkyle en $C_1$ à $C_8$ ou d'un sel pharmacologiquement acceptable de ce composé, et d'un analgésique narcotique.

3. Composition suivant la revendication 2, qui comprend en outre un excipient physiologiquement acceptable.

4. Produit suivant la revendication 1, dans lequel l'analgésique narcotique est choisi entre la morphine, l'hydromorphone, l'oxymorphone, le lévorphanol, la méthadone, la mépéridine, l'aniléridine, l'alphaprodine, le fentanol, la codéine, la codone et l'oxycodone.

5. Composition suivant la revendication 2 ou la revendication 3, dans laquelle l'analgésique narcotique est choisi entre ceux qui sont définis dans la revendication 4.